# EUROPÄISCHE PATENTSCHRIFT

(11) **EP 1 854 453 B1**
(45) Veröffentlichungstag und Bekanntmachung des Hinweises auf die Patenterteilung: **16.02.2011**
(21) Anmeldenummer: 07008906.5
(22) Anmeldetag: 02.05.2007
(51) Int. Cl.: A61K 9/00, A61K 31/205, A61K 33/06, A61K 31/198, A23L 1/29, A61K 9/14

(54) **Lagerstabile wässrige Zusammensetzungen, umfassend eine Magnesiumverbindung und L-Carnitin**
Aqueous composition with shelf life stability comprising a magnesium compound and L-carnitine
Composition aqueuse avec stabilité au stockage comprenant une composé du magnesium et du L-carnitine

(30) Priorität: 12.05.2006 EP 06009865
(43) Veröffentlichungstag der Anmeldung: 14.11.2007
(60) Teilanmeldung: 10004937.8
(73) Patentinhaber: Verla-Pharm Arzneimittelfabrik Apotheker H.J. von Ehrlich GmbH & Co. KG, D-82327 Tutzing (DE)
(72) Erfinder: Hopf, Günter, 82327 Tutzing (DE)
(74) Vertreter: Müller-Boré & Partner Patentanwälte

(56) Entgegenhaltungen:
- EP-A1- 1 163 904
- EP-A2- 0 402 755
- EP-A2- 0 680 945
- WO-A-01/52647
- WO-A-01/91759
- US-A1- 2002 132 219

## Beschreibung

Die vorliegende Erfindung betrifft lagerstabile wässrige bzw. gelartige Zusammensetzungen, umfassend Magnesium-L-aspartat-Hydrochlorid und L-Carnitin, und deren Verwendung zur Herstellung eines Arzneimittels zur Unterstützung des Stoffwechsels, insbesondere im Muskelgewebe, und des Muskelaufbaus bzw. deren Verwendung als Nahrungsergänzungsmittel oder im Veterinärbereich als Tierfutterzusatz, insbesondere für Pferde, Geflügel, Tauben, Schweine, Rinder, Schafe und Kamele.

Im Stand der Technik sind wässrige Magnesium-enthaltende Lösungen (beispielsweise Nupafeed®, vertrieben von Verla-Pharm) bekannt, die etwa 50 Gew.-% Magnesiumaspartathydrochlorid enthalten. Diese und höhere Magnesiumkonzentrationen sind bei derartigen Lösungen aufgrund der zunehmenden Kristallisationsneigung der enthaltenen Magnesiumverbindung aber problematisch. Demgemäß kann keine über einen längeren Zeitraum lagerstabile wässrige Lösung mit einem hohen Gehalt an Magnesiumverbindung erhalten werden.

EP 0 680 945 A2 offenbart wässrige Zusammensetzungen mit Carnitin und Magnesiumchlorid.

Die der vorliegenden Erfindung zugrundeliegende technische Aufgabe besteht somit darin, eine Magnesiumionen-enthaltende Lösung bereitzustellen, welche über einen langen Zeitraum lagerstabil ist, insbesondere eine hohe Magnesiumkonzentration aufweist, und kein unerwünschtes Auskristallisieren der verwendeten Magnesiumverbindung oder anderer Komponenten zeigt.

Diese Aufgabe wird durch Bereitstellen der in den Ansprüchen gekennzeichneten Ausführungsformen gelöst.

Gemäß der vorliegenden Erfindung wird eine wässrige Zusammensetzung bzw. gelartige Zusammensetzung bereitgestellt, umfassend die Magnesiumkomplexverbindung Magnesium-L-aspartat-Hydrochlorid mit i einem Gehalt in einem Bereich von 50 bis 75 Gew.% und L-Carnitin mit einem Gehalt von mindestens 5 Gew.-%.

Die in der vorliegenden Erfindung verwendete Magnesiumkomplexverbindung ist ausreichend gut in Wasser löslich. Ferner weist die Magnesiumkomplexverbindung eine gute Bioverfügbarkeit und ein günstiges Freisetzungsverhalten im menschlichen und tierischen Organismus auf.

Gemäß der vorliegenden Erfindung umfaßt die wässrige bzw. gelartige Zusammensetzung 50 bis 75 Gew.-%, mehr bevorzugt 50 bis 70 Gew.-% der Magnesiumkomplexverbindung und mindestens 5 Gew.-% L-Carnitin.

L-Carnitin bzw. 3-Hydroxy-4-(trimethylammonio)-buttersäurebetain ist eine farblose, gut wasserlösliche, hygroskopische Substanz, die ein charakteristischer Bestandteil der gestreiften Muskulatur ist und vor allem im Fettsäurestoffwechsel als Carrier für Acylgruppen durch die Mitochondrien-Membran hindurch dient.

Die erfindungsgemäße Zusammensetzung enthält L-Carnitin, da durch Zugabe von L-Carnitin die Kristallisation von Magnesiumsalzen bzw. Magnesiumkomplexverbindungen selbst in hochkonzentrierten Lösungen wirkungsvoll verhindert oder zumindest deutlich verzögert werden kann. Besonders bevorzugt liegt der Gehalt an L-Carnitin, bezogen auf die Gesamtzusammensetzung, in einem Bereich von etwa 5 bis etwa 35 Gew.-%, wobei ein Bereich von 7 bis 30 Gew.-% am meisten bevorzugt ist.

Überraschenderweise wurde festgestellt, daß selbst bei einer sehr geringen Konzentration von L-Carnitin in der erfindungsgemäßen wässrigen Lösung die problematische Kristallisation eines Magnesiumsalzes und/oder einer Magnesiumkomplexverbindung wirkungsvoll verhindert bzw. zumindest deutlich verzögert werden kann. Beispielsweise ist es möglich, eine stabile wässrige Lösung mit 70 Gew.-% Magnesiumaspartathydrochlorid zu erhalten, wenn 5 Gew.-% L-Carnitin in der Lösung vorhanden sind. Der kristallisationshemmende Effekt läßt sich sogar noch weiter steigern, wenn in der vorstehenden Lösung 15 Gew.-% L-Carnitin vorhanden sind.

Der Gehalt der Magnesiumkomplexverbindung, bezogen auf die Gesamtzusammensetzung, beträgt mindestens 50 Gew.-%, wobei auch bei ≥ 60 Gew.-% und sogar bei ≥ 70 Gew.-% lagerstabile wässrige Lösungen erhalten werden, die keine oder eine stark verzögerte Kristallisationsneigung zeigen, wenn ≥ 5 Gew.-%, vorzugsweise ≥ 7 Gew.-%, L-Carnitin vorhanden ist. Der Gehalt der Magnesiumkomplexverbindung, bezogen auf die Gesamtzusammensetzung, liegt in einem Bereich von 50 bis 75 Gew.-%, wobei ein Gehalt in einem Bereich von 50 bis 70 Gew.-% am meisten bevorzugt ist.

Die erfindungsgemäße wässrige Zusammensetzung kann weiterhin übliche Hilfsstoffe, wie Stabilisierungsmittel, Aromastoffe, Konservierungsmittel, Arzneistoffe oder Farbstoffe, enthalten. Gemäß einer bevorzugten Ausführungsform ist in der erfindungsgemäßen wässrigen Zusammensetzung Kaliumsorbat enthalten, wobei ein Gehalt von etwa 0,05 bis 0,2 Gew.-% Kaliumsorbat besonders bevorzugt ist.

Die erfindungsgemäße wässrige Zusammensetzung kann durch einfaches Vermischen von Wasser, der Magnesiumkomplexverbindung, L-Carnitin und gegebenenfalls einem oder mehreren Hilfsstoffen hergestellt werden. Es ist jedoch bevorzugt, die erfindungsgemäße wässrige Zusammensetzung durch Lösen von L-Carnitin in einer zuvor gebildeten Lösung aus Wasser, der Magnesiumkomplexverbindung und gegebenenfalls einem oder mehreren Hilfsstoffen herzustellen.

Die erfindungsgemäße wässrige bzw. gelartige Zusammensetzung kann auch in ein entsprechendes Sprühtrocknungsprodukt umgewandelt werden, welches durch Sprühtrocknen einer wässrigen Zusammensetzung gemäß der vorliegenden Erfindung erhalten wird.

Darüber hinaus wird gemäß der vorliegenden Erfindung die Verwendung der vorstehend beschriebenen wässrigen Zusammensetzung oder des vorstehend beschriebenen Sprühtrocknungsprodukts im Humanbereich als Arzneimittel bzw. als Nahrungsergänzungsmittel oder im Veterinärbereich als Tierfutterzusatz, insbesondere für Pferde, Geflügel, Tauben, Schweine, Rinder, Schafe und Kamele, bereitgestellt.

Die erfindungsgemäße wässrige Zusammensetzung eignet sich besonders gut zur Verabreichung an Pferde oder Kamele durch einen sogenannten Maulinjektor. Durch Verwendung der erfindungsgemäßen Zusammensetzung kann das unerwünschte Blockieren des Kolbens in einem Maulinjektor durch Auskristallisieren der enthaltenen Magnesiumverbindung wirkungsvoll verhindert werden.

Das in der erfindungsgemäßen Zusammensetzung eingesetzte L-Carnitin weist somit nicht nur den überraschenden Effekt der Kristallisationshemmung bzw. Stabilisierung der wässrigen Lösung auf, sondern bietet in seiner Eigenschaft, den Stoffwechsel, insbesondere im Muskelgewebe, und den Muskelaufbau bzw. die Leistungssteigerung bei Mensch und Tier zu unterstützen, beim Einsatz der erfindungsgemäßen Zusammensetzung als Arzneimittel, Nahrungsergänzungsmittel oder als Tierfutterzusatz weitere vorteilhafte Eigenschaften.

Die nachfolgenden Beispiele werden angegeben, um die vorliegenden Erfindung weiter zu erläutern, ohne diese darauf zu beschränken.

### Beispiele

### Beispiel 1 (insbesondere als Veterinärprodukt) (nicht gemäß der Erfindung)

Ein lagerstabiles wässriges Gel, das keine Kristallisationsneigung bei Lagerung über mehrere Wochen bei 20°C zeigte, wurde erhalten, wenn die nachfolgenden Bestandteile vermischt wurden:
43,35 g Magnesium-L-aspartathydrochlorid
0,05 g Kaliumsorbat
30,24 g Wasser
26,36 g L-Carnitin

### Beispiel 2 (insbesondere als Veterinärprodukt) (nicht gemäß der Erfindung)

Ein lagerstabiles wässriges Gel, das keine Kristallisationsneigung bei Lagerung über mehrere Wochen bei 20°C zeigte, wurde erhalten, wenn die nachfolgenden Bestandteile vermischt wurden:
49,84 g Magnesium-L-aspartathydrochlorid
0,05 g Kaliumsorbat
20,00 g Wasser
30,10 g L-Carnitin

### Beispiel 3 (insbesondere als Veterinärprodukt) (nicht gemäß der Erfindung)

Eine lagerstabile wässrige Lösung, die keine Kristallisationsneigung bei Lagerung über mehrere Wochen bei 20°C zeigte, wurde erhalten, wenn die nachfolgenden Bestandteile vermischt wurden:
28,13 g Magnesium-L-aspartathydrochlorid
0,029 g Kaliumsorbat
54,86 g Wasser
16,99 g L-Carnitin

### Beispiel 4 (insbesondere als Humanprodukt) (nicht gemäß der Erfindung)

Ein lagerstabiles wässriges Gel, das keine Kristallisationsneigung bei Lagerung über mehrere Wochen bei 20°C zeigte, wurde erhalten, wenn die nachfolgenden Bestandteile vermischt wurden:
60,51 g Magnesium-L-aspartathydrochlorid
0,07 g Kaliumsorbat.
29,93 g Wasser
9,49 g L-Carnitin

### Beispiel 5 (insbesondere als Humanprodukt)

Eine lagerstabile wässrige Lösung, die keine Kristallisationsneigung bei Lagerung über mehrere Wochen bei 20°C zeigte, wurde erhalten, wenn die nachfolgenden Bestandteile vermischt wurden:
43,22 g Magnesium-L-aspartathydrochlorid
0,07 g Kaliumsorbat
49,93 g Wasser
6,78 g L-Carnitin

### Beispiel 6 (nicht gemäß der Erfindung)

Eine lagerstabile wässrige gelartige Lösung, die keine Kristallisationsneigung bei Lagerung über mehrere Wochen bei 20°C zeigte, wurde erhalten, wenn die nachfolgenden Bestandteile vermischt wurden:
51,2 g Magnesium-L-aspartathydrochlorid
0,1 g Kaliumsorbat
48,7 g Wasser
100 g L-Carnitin

### Beispiel 7 (nicht gemäß der Erfindung)

Eine lagerstabile wässrige gelartige Lösung, die keine Kristallisationsneigung bei Lagerung über mehrere Wochen bei 20°C zeigte, wurde erhalten, wenn die nachfolgenden Bestandteile vermischt wurden:
51,2 g Magnesium-L-aspartathydrochlorid
0,1 g Kaliumsorbat
48,7 g Wasser
150 g L-Carnitin

### Vergleichsbeispiel 1

Eine nicht lagerstabile wässrige Lösung, die nach wenigen Tagen einen kristallinen Niederschlag von Magnesium-L-aspartathydrochlorid zeigte, wurde erhalten, wenn die nachfolgenden Bestandteile vermischt wurden:
70 g Magnesium-L-aspartathydrochlorid
0,1 g Kaliumsorbat
30 g Wasser

## Patentansprüche

1. Wässrige bzw. gelartige Zusammensetzung, umfassend die Magnesiumkomplexverbindung Magnesium-L-aspartat-Hydrochlorid mit einem Gehalt in einem Bereich von 50 bis 75 Gew.% und L-Carnitin mit einem Gehalt von mindestens 5 Gew.-%.

2. Zusammensetzung nach Anspruch 1, wobei der Gehalt an L-Carnitin in einem Bereich von 5 bis 35 Gew.% liegt.

3. Zusammensetzung nach Anspruch 1 oder 2, weiter umfassend ein oder mehrere Hilfsstoffe.

4. Verwendung einer Zusammensetzung nach einem der Ansprüche 1 bis 3 als Nahrungsergänzungsmittel oder als Tierfutterzusatz.

5. Verwendung einer Zusammensetzung nach einem der Ansprüche 1 bis 3 zur Herstellung eines Arzneimittels zur Unterstützung des Stoffwechsels, insbesondere im Muskelgewebe, und des Muskelaufbaus.

## Claims

1. An aqueous or gel-like composition comprising the magnesium complex compound magnesium L-aspartate hydrochloride in an amount in the range of 50% by weight to 75% by weight and L-carnitine in an amount of at least 5% by weight.

2. The aqueous composition according to Claim 1, wherein L-carnitine is present in an amount ranging from 5% by weight to 35% by weight.

3. The aqueous composition according to Claim 1 or 2, further comprising one or more auxiliaries.

4. Use of an aqueous composition according to one of Claims 1 to 3 as food supplement, or as animal feed additive.

5. Use of an aqueous composition according to one of Claims 1 to 3 for the preparation of a medicament for supporting metabolism, in particular in muscle tissue, and for supporting muscle structure.

## Revendications

1. Composition aqueuse ou de type gel, comprenant le composé de complexe de magnésium, chlorhydrate de L-aspartate - magnésium présentant une teneur dans une plage de 50 à 75 % en poids et de la L-carnitine à une teneur d'au moins 5 % en poids.

2. Composition selon la revendication 1, dans laquelle la teneur en L-carnitine se situe dans une plage de 5 à 35 % en poids.

3. Composition selon la revendication 1 ou 2, comprenant en outre un ou plusieurs adjuvants.

4. Utilisation d'une composition selon l'une des revendications 1 à 3, comme complément alimentaire ou comme additif alimentaire pour animaux.

5. Utilisation d'une composition selon l'une des revendications 1 à 3, pour la production d'un médicament pour stimuler le métabolisme, en particulier dans les tissus musculaires et pour la constitution musculaire.
